# EUROPEAN PATENT SPECIFICATION

(11) **EP 1 781 272 B1**
(45) Date of publication and mention of the grant of the patent: **06.09.2017**
(21) Application number: 05769972.0
(22) Date of filing: 25.07.2005
(51) Int. Cl.: A61K 31/13, A61P 29/00, A61P 25/00, A61K 31/7125

(54) **USE OF COMPOUNDS ACTIVE ON THE SIGMA RECEPTOR FOR THE TREATMENT OF MECHANICAL ALLODYNIA**
VERWENDUNG VON VERBINDUNGEN MIT WIRKUNG AUF DEN SIGMA-REZEPTOR ZUR BEHANDLUNG VON MECHANISCHER ALLODYNIE
UTILISATION DE COMPOSES ACTIFS SUR LE RECEPTEUR SIGMA POUR LE TRAITEMENT DE L'ALLODYNIE MECANIQUE

(30) Priority: 24.07.2004 EP 04017561; 24.07.2004 EP 04017562; 30.07.2004 US 902273; 30.07.2004 US 902272; 27.08.2004 EP 04020376
(43) Date of publication of application: 09.05.2007
(73) Proprietor: LABORATORIOS DEL DR. ESTEVE, S.A., 08041 Barcelona (ES)
(72) Inventor: BAEYENS CABRERA, José M., 18016 Granada (ES)
(74) Representative: Peters, Hajo
(86) International application number: PCT/EP2005/008080
(87) International publication number: WO 2006/010587

(56) References cited:
- EP-B- 1 244 498
- WO-A-03/078437
- US-A1- 2003 171 347
- US-B1- 6 596 756
- SJOGREN, P. ET AL.: "Disappearance of morphine-induced hyperalgesia after discontinuing or substituting morphine with other opioid agonists" PAIN, vol. 59, 1994, pages 313-316, XP002356374
- STEWART, L.S.A. & MARTIN, W.J.: "Influence of positive analgesics on development of neuropathic pain in rats" COMPARATIVE MEDICINE, vol. 53, no. 1, February 2003 (2003-02), pages 29-36, XP009057765
- GAGNON, B. ET AL.: "Methadone in the treatment of neuropathic pain", PAIN RES MANAGE, vol. 8, no. 3, 2003, pages 149-154, XP009173229,
- Laurent Monassier ET AL: "R Sigma receptors: from discovery to highlights of their implications in the cardiovascular system", , 1 January 2002 (2002-01-01), XP55187526, Retrieved from the Internet: URL:http://onlinelibrary.wiley.com/doi/10. 1046/j.1472-8206.2002.00063.x/abstract;jse ssionid=D7ACF5CE06F742016C5E60EB9EB828B2.f 02t04 [retrieved on 2015-05-05]

## Description

### Field of the invention

The present invention refers to the use of compounds active on the sigma receptor for the treatment of the symptoms of mechanical allodynia, as well as treatment of the disease causing the symptoms, the prevention or the prophylaxis of the symptoms of mechanic allodynia, as well as the prevention or the prophylaxis of the disease causing the symptoms.

### Background of the invention

The treatment of pain conditions is of great importance in medicine. There is currently a world-wide need for additional pain therapy. The pressing requirement for a specific treatment of pain conditions or as well a treatment of specific pain conditions which is right for the patient, which is to be understood as the successful and satisfactory treatment of pain for the patients, is documented in the large number of scientific works which have recently and over the years appeared in the field of applied analgesics or on basic research on nociception.

PAIN is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210). Even though pain is always subjective its causes or syndromes can be classified.

Especially mechanical allodynia which in the past years has developed into a major health problem in broad areas of the population needs a very specific treatment, especially considering that any treatment of mechanical allodynia is extremely sensitive to the causes behind the pain, be it the disease ultimately causing it or the mechanistic pathway over which it develops.

Therefore, it was the underlying problem solved by this invention to find new ways of treating mechanical allodynia.

So, the main object of this invention is the use of a compound binding to the sigma receptor in the production of a medicament for the treatment of mechanical allodynia.

In a first aspect the invention thus refers to the use of at least one compound binding to the sigma receptor and having an IC₅₀ value of ≤50 nM for the production of a medicament for the treatment of mechanical allodynia, characterized in that said compound binding to the sigma receptor is acting on the sigma receptor as an antagonist, characterized in that said compound binding to the sigma receptor is binding to the sigma-1 receptor subtype.

This/these compound/s may be in neutral form, the form of a base or acid, in the form of a salt, preferably a physiologically acceptable salt, in the form of a solvate or of a polymorph and/or in the form of in the form of its racemate, pure stereoisomers, especially enantiomers or diastereomers or in the form of mixtures of stereoisomers, especially enantiomers or diastereomers, in any suitable mixing ratio.

While working on compounds binding to the sigma receptor and with models like knock-out mice it was surprisingly found out that mechanical allodynia is connected to the sigma receptor so that compounds binding to the sigma receptor were acting on mechanical allodynia with a high patency.

"Treating" or "treatment" as used in this application are defined as including the treatment of the symptoms - of mechanical allodynia - as well as treatment of the disease or disease consequences causing the symptoms, the prevention or the prophylaxis of the symptoms - of mechanical allodynia - as well as the prevention or the prophylaxis of the disease or disease consequences causing the symptoms. Preferably "treating" or "treatment" as used in this application are defined as including the treatment of the symptoms - of mechanical allodynia - as well as treatment of the disease consequences causing the symptoms, the prevention or the prophylaxis of the symptoms - of mechanical allodynia - as well as the prevention or the prophylaxis of the disease consequences causing the symptoms. Most preferably "treating" or "treatment" as used in this application are defined as including the treatment of the symptoms of mechanical allodynia, and the prevention or the prophylaxis of the symptoms of mechanical allodynia.

"The sigma receptor/s" as used in this application is/are well known and defined using the following citation: This binding site represents a typical protein different from opioid, NMDA, dopaminergic, and other known neurotransmitter or hormone receptor families (G. Ronsisvalle et al. Pure Appl. Chem. 73, 1499-1509 (2001)). Pharmacological data based on ligand binding studies, anatomical distribution and biochemical features distinguish at least two subtypes of σ receptors (R. Quiron et al., Trends Pharmacol. Sci. 13, 85-86 (1992); M.L.Leitner, Eur. J. Pharmacol. 259, 65-69 (1994); S.B. Hellewell and W.D. Bowen; Brain Res. 527, 244-253 (1990)) (G. Ronsisvalle et al. Pure Appl. Chem. 73, 1499-1509 (2001)). The protein sequence of sigma receptors (Sigma 1 (σ1) and Sigma 2 (σ2)) is known (e.g. Prasad, P.D. et al., J. Neurochem. 70 (2), 443-451 (1998)) and they show a very high affinity for e.g. pentazocine.

"Compound/s binding to the sigma receptor" or "sigma ligand" as used in this application is/are defined as having an IC₅₀ value of ≤ 5000 nM (non-claimed), more preferably ≤ 1000 nM (non-claimed), more preferably ≤ 500 nM (non-claimed). More preferably, the IC₅₀ value is ≤ 250 nM (non-claimed). More preferably, the IC₅₀ value is ≤ 100 nM (non-claimed). Most preferably, the IC₅₀ value is ≤ 50 nM. Additionally, the wording "Compound/s binding to the sigma receptor", as used in the present application is defined as having at least >50% displacement using 10 mM radioligand specific for the sigma receptor (e.g. preferably ¹H-pentazocine) whereby the sigma recepor is the sigma-1 receptor subtype.

Compounds binding to the sigma receptor generally also known as sigma ligands are well known in the art with many of them falling under the definition for "Compound/s binding to the sigma receptor" set up above. Still even though there are many uses known for sigma ligands such as antipsychotic drugs, anxiolytics, antidepressants, the treatment of stroke, antiepileptic drugs and many other indications including anti-migraine and general pain (mostly analgesia) there is nowhere any mentioning of these compounds being useful against mechanical allodynia.

Preferably, compounds selected from the group consisting of amitriptyline, desipramine, fluoxetine, methadone and tiagabine, are disclaimed from the present invention. These compounds have been shown to bind to the sigma receptor and have an IC₅₀ value ≥ 100 nM.

Preferably, compounds selected from the group consisting of agmatine, alfentanil, all-trans retinoic acid (ATRA), Erythropoietin, Etanercept, GV196771, GV196771A, GW406381X, KRN5500, L-N (6)-(1-iminoethyl) lysine (L-NIL), LY379268, LY389795, neurotropin, N-methyl-D-aspartic acid, peptide analog of thymulin (PAT), Propentofylline, ReN-1869 [(R)-1-(3-(10,11-dihydro-5H-dibenzo[a,d]cyclohepten-5-ylidene)-1- propyl)-3-piperidine carboxylic acid], R-phenylisopropyl-adenosine (R-PIA), SD-282, Semaphorin3A, SH9119, T62 (2-amino-3-(4-chlorobenzoyl)-5,6,7,8-tetrahydrobenzothiophene) and ziconotide are disclaimed from the present invention. The majority of these compounds has an IC₅₀ ≥ 100 nM with respect to binding the sigma receptor.

Compounds binding to the sigma receptor known in the art and matching the criteria of sigma ligand (i.e. having an IC₅₀ ≤ 5000 nM) as mentioned above, are listed below. Some of these compounds may bind to the sigma-1 and/or the sigma-2 receptor. Preferably, these compounds are in form of a salt, a base or an acid. Also preferably, the salts/bases/acids indicated in the list are to be understood as being exemplary and therefore may respresent any salt, base or acid of the compound.

| | |
|---|---|
| 4-(4-Fluorobenzoyl)-1-(4-Phenylbutyl)Piperidine Oxalate | 4'-Chloro-3-Alpha-(Diphenylmethoxy)Tropane HCl |
| 4-Furan-2-Ylmethyl-Piperazine-1-Carboxylic Acid 2-{4-(3-(2-Trifluoromethyl-Phenothiazin-10-Yl)-Propyl)-Piperazin-Yl}-Ethyl Ester | Astemizole |
| BD 1008 DiHBr | Benproperine Phosphate |
| BD 1047 | NE 100 |
| BD 1063 | Bromperidol |
| Bromhexine HCl | HEAT HCl |
| Cis-(+/-)-N-Methyl-N-[2-(3, 4-Dichlorophenyl)Ethyl]-2-(1-Pyrrolidinyl)Cyclohexamine DiHBr | GBR 12909 DiHCl |
| GBR-12935 DiHCl | Ifenprodil Tartrate |
| L-693,403 Maleate | Iomerizine HCl |
| MR 16728 HCl | L-741,742 HCl |
| PAPP | Lobeline HCl |
| RS 67333 HCl | Trimeprazine Hemi-L-Tartrate |
| Trifluperidol HCl | |

Another aspect of the present invention relates to BD-1063 and its derivatives, a compound binding to the sigma receptor, and its use for the production of a medicament for the treatment of mechanical allodynia.

The synthesis of BD-1063 and compounds structurally related (mostly those covered under formula IB) is described in detail in de Costa et al. (1993), J. Med. Chem. 36(16): 2311-2320 (referred to as compound 4 in Scheme I; p. 2312) Accordingly the synthesis of the compound whose use is claimed in this invention is known and the compound thus is available to those skilled in the art, starting from the given information working analogously if necessary.

Compounds according to general formula IB have been shown to be sigma ligands according to the above mentioned definition.

Therefore, another aspect of the present invention is the use of a compound of general formula IB 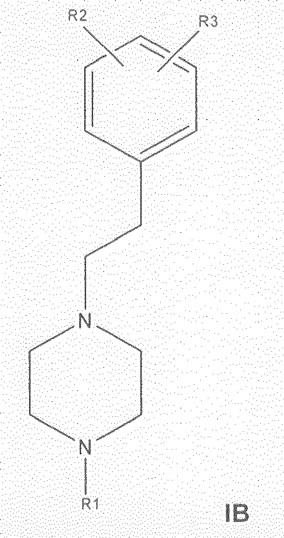 wherein
R1 is selected from C₁₋₆-Alkyl, saturated or unsaturated, substituted or not substituted, branched or not branched;
R2 and R3 are independently of each other selected from H; C₁₋₆Alkyl, saturated or unsaturated, substituted or not substituted, branched or not branched; Halogen, O-C₁₋₆-Alkyl, saturated or unsaturated, substituted or not substituted, branched or not branched;
in the production of a drug to treat mechanical allodynia.

In a preferred embodiment of the invention the compound used is a compounds according to general formula IB, wherein R1 is selected from C₁₋₆-Alkyl, saturated or unsaturated, branched or not branched, unsubstituted or substituted with F, Cl, Br, I, NH₂, SH or OH.

In a preferred embodiment of the invention the compound used is a compound according to general formula IB, wherein R2 and R3 are independently of each other selected from H; C₁₋₆-Alkyl, saturated or unsaturated, branched or not branched, unsubstituted or substituted with F, Cl, Br, I, NH₂, SH or OH; Halogen; O-C₁₋₆-Alkyl, saturated or unsaturated, branched or not branched, unsubstituted or substituted with F, Cl, Br, I, NH₂, SH or OH.

In a preferred embodiment of the invention the compound used is a compound according to general formula IB, wherein
R¹ is selected from C₁₋₄-Alkyl, saturated or unsaturated, substituted or not substituted, and branched or not branched;
preferably R¹ is selected from C₁₋₄-Alkyl, saturated, substituted or not substituted, and branched or not branched;
more preferably R¹ is selected from C₁₋₄-Alkyl, saturated, not substituted, and branched or not branched; namely CH₃, C₂H₅, C₃H₇, C₄H₉.

In a preferred embodiment of the invention the compound used is a compound according to general formula IB, wherein
R² and R³ are independently of each other selected from H; OH, SH, NH₂, C₁₋₄-Alkyl, saturated or unsaturated, substituted or not substituted, branched or not branched; F, Cl, Br, I; O-C₁₋₄-Alkyl, saturated or unsaturated, substituted or not substituted, branched or not branched;
preferably R² and R³ are independently of each other selected from H; OH, NH₂, C₁₋₄-Alkyl, saturated, substituted or not substituted, and branched or not branched; F, Cl, Br, I; O-C₁₋₄-Alkyl, saturated, not substituted, and branched or not branched.

In a preferred embodiment of the invention the compound used is a compound according to general formula IB, wherein
R² and R³ are independently of each other selected from H, OH, NH₂, CH₃, C₂H₅, C₃H₇, C₄H₉, CF₃, CHF₂, Cl, F, Br, I, O-CH₃, O-C₂H₅, O-C₃H₇, O-C₄H₉;
preferably R² and R³ are independently of each other selected from H, F, Cl and CF₃.

Here it is also preferred if R² and R³ are in 3' and 4' position on the phenyl ring.

In a highly preferred embodiment of the invention the compound according to general formula IB used is 1-(3,4-dichlorophenethyl)-4-methylpiperazine, optionally in the form of its racemate, pure stereoisomers, especially enantiomers or diastereomers or in the form of mixtures of stereoisomers, especially enantiomers or diastereomers, in any suitable ratio; in the form described or in form of an acid or base or in form of a salt, especially a physiologically acceptable salt, or in form of a solvate, especially a hydrate.
Unless otherwise stated, the compound of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

Another aspect of the present invention relates to BD-1047, a compound binding to the sigma receptor, and its use for the production of a medicament for the treatment of mechanical allodynia.

BD-1047 (N1-(3,4-dichlorophenethyl)-N1,N2,N2-trimethylethane-1,2-diamine/ N-[2-(3,4-dichlorophenyl)ethyl]-N-methyl-2-(dimethylamino)-ethylamine) is, like BD-1063, commercially available and also a popular tool compound as sigma receptor ligands (Kᵢ = 0.9 nM for stigma-1). As according to US2003/0171347A1, BD-1047 shows no or only slight binding to a selection of other relevant receptors (page 7, table2 and page 8, table 3).
The synthesis of BD-1047 is described in detail in de Costa et al. (1992), J. Med Chem. 35, 38-47, as well as WO92/22279 A1 showing compounds related to CNS disorders, where BD-1047 is described as a truncated version (compound 10 in Scheme 2, p.17).

Another aspect of the disclosure is the use of BD-1047 and/or at least one compound of general formula IB as defined above for the production of a medicament for the treatment of neuropathic pain.

In the context of the whole invention, alkyl and cycloalkyl radicals are understood as meaning saturated and unsaturated (but not aromatic), branched, unbranched and cyclic hydrocarbons, which can be unsubstituted or mono- or polysubstituted. In these radicals, C₁₋₂-alkyl represents C1- or C2-alkyl, C₁₋₃-alkyl represents C1-, C2- or C₃₋alkyl, C₁₋₄-alkyl represents C1-, C2-, C3- or C4-alkyl, C₁₋₅-alkyl represents C1-, C2-, C3-, C4-, or C5-alkyl, C₁₋₆-alkyl represents C1-, C2-, C3-, C4-, C5- or C6-alkyl, C₁₋₇-alkyl represents C1-, C2-, C3-, C4-, C5-, C6- or C7-alkyl, C₁₋₈-alkyl represents C1-, C2-, C3-, C4-, C5-, C6-, C7- or C8-alkyl, C₁₋₁₀-alkyl represents C1-, C2-, C3-, C4-, C5-, C6-, C7-, C8-, C9- or C10-alkyl and C₁₋₁₈-alkyl represents C1-, C2-, C3-, C4-, C5-, C6-, C7-, C8-, C9-, C10-, C11-, C12-, C13-, C14-, C15-, C16-, C17- or C18-alkyl. Furthermore, C₃₋₄-cycloalkyl represents C3- or C4-cycloalkyl, C₃₋₅-cycloalkyls represents C3-, C4- or C5-cycloalkyl, C₃₋₆-cycloalkyl represents C3-, C4-, C5- or C6-cycloalkyl, C₃₋₇-cycloalkyl represents C3-, C4-, C5-, C6- or C7-cycloalkyl, C₃₋₈-cycloalkyl represents C3-, C4-, C5-, C6-, C7- or C8-cycloalkyl, C₄₋₅-cycloalkyl represents C4- or C₅-cycloalkyl, C₄₋₆-cycloalkyl represents C4-, C5- or C6-cycloalkyl, C₄₋₇-cycloalkyl represents C4-, C5-, C6- or C7-cycloalkyl, C₅₋₆-cycloalkyl represents C5- or C6-cycloalkyl and C₅₋₇-cycloalkyl represents C5-, C6- or C7-cycloalkyl. In respect of cycloalkyl, the term also includes saturated cycloalkyls in which one or 2 carbon atoms are replaced by a heteroatom, S, N or O. However, mono- or polyunsaturated, preferably monounsaturated, cycloalkyls without a heteroatom in the ring also in particular fall under the term cycloalkyl as long as the cycloalkyl is not an aromatic system. The alkyl and cycloalkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, cyclopropyl, 2-methylcyclopropyl, cyclopropylmethyl, cyclobutyl, cyclopentyl, cyclopentylmethyl, cyclohexyl, cycloheptyl, cyclooctyl, and also adamantyl, (if substituted also CHF₂, CF₃ or CH₂OH) as well as pyrazolinone, oxopyrazolinone, [1,4]-dioxane or dioxolane.

In the context of the whole invention, in connection with alkyl and cycloalkyl - unless expressly defined otherwise - the term substituted in the context of this invention is understood as meaning replacement of at least one hydrogen radical by F, Cl, Br, I, NH₂, SH or OH, "polysubstituted" radicals being understood as meaning that the replacement takes effect both on different and on the same atoms several times with the same or different substituents, for example three times on the same C atom, as in the case of CF₃, or at different places, as in the case of -CH(OH)-CH=CH-CHCl₂. Particularly preferred substituents here are F, Cl and OH. In respect of cycloalkyl, the hydrogen radical can also be replaced by OC₁₋₃-alkyl or C₁₋₃-alkyl (in each case mono- or polysubstituted or unsubstituted), in particular methyl, ethyl, n-propyl, i-propyl, CF₃, methoxy or ethoxy.

In a preferred embodiment of the present invention, with respect to compounds of general formula IB, R1, R2 and/or R3 are at least optionally substituted with F, Cl, Br, I, NH₂, SH or OH.

Referring to the whole invention, the term (CH₂)₃₋₆ is to be understood as meaning - CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning -CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and CH₂-CH₂-CH₂-CH₂-CH₂-, etc.

An aryl radical is understood in the whole invention as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

Additionally, in the context of the whole invention, a heteroaryl radical is understood as meaning heterocyclic ring systems which have at least one unsaturated ring and can contain one or more heteroatoms from the group consisting of nitrogen, oxygen and/or sulfur and can also be mono- or polysubstituted. Examples which may be mentioned from the group of heteroaryls are furan, benzofuran, thiophene, benzothiophene, pyrrole, pyridine, pyrimidine, pyrazine, quinoline, isoquinoline, phthalazine, benzo-1,2,5-thiadiazole, benzothiazole, indole, benzotriazole, benzodioxolane, benzodioxane, carbazole and quinazoline.

In addition, referring to the whole invention, in connection with aryl and heteroaryl, substituted is understood as meaning substitution of the aryl or heteroaryl by R, OR, a halogen, preferably F and/or Cl, a CF₃, a CN, an NO₂, an NRR, a C₁₋₆-alkyl (saturated), a C₁₋₆-alkoxy, a C₃₋₈-cycloalkoxy, a C₃₋₈-cycloalkyl or a C₂₋₆-alkylene.

The term "salt" is to be understood as meaning any form of the active compound according to the invention in which this assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

According to the IASP "allodynia" is defined as "a pain due to a stimulus which does not normally provoke pain" (IASP, Classification of chronic pain, 2nd Edition, IASP Press (2002), 210). Even though the symptoms of allodynia are most likely associated as symptoms of neuropathic pain this is not necessarily the case so that there are symptoms of allodynia not connected to neuropathic pain though rendering mechanical allodynia in some areas broader then neuropathic pain.

Mechanical allodynia is a form of allodynia where mechanical stimuli cause the painful sensation in contrast to thermal alladynia where the painful sensation comes from a thermal stimulus (e.g. heat).

"Neuropathic pain" is defined by the IASP as "pain initiated or caused by a primary lesion or dysfunction in the nervous system" (IASP, Classification of chronic pain, 2nd Edition, IASP Press (2002), 210). For the purpose of this invention included under this heading or to be treated as synonymous is "Neurogenic Pain" which is defined by the IASP as "pain initiated or caused by a primary lesion, dysfunction or transitory perturbation in the peripheral or central nervous system".

The IASP draws the following difference between "allodynia," "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

In another preferred embodiment of the use according to the invention the mechanical allodynia derived from central pain.

In another preferred embodiment of the use according to the invention the mechanical allodynia derived from peripheral pain.

In another preferred embodiment of the invention the mechanical allodynia is derived from neuropathic pain.

In a very preferred embodiment of the invention the compound binding to the sigma receptor used is acting on the sigma receptor as an antagonist.

In a very preferred embodiment of the invention the compound binding to the sigma receptor used is acting on the sigma receptor as an inverse agonist.

In a very preferred embodiment of the invention the compound binding to the sigma receptor used is acting on the sigma receptor as a partial antagonist.

In another possible embodiment of the invention (non-claimed) the compound binding to the sigma receptor used is acting on the sigma receptor as an agonist.

In another embodiment of the invention the compound binding to the sigma receptor used is acting on the sigma receptor as a mixed agonist/antagonist, a partial agonist or a partial antagonist (non-claimed).

In another embodiment of the invention the sigma receptor to which the "compound binding to the sigma receptor" is binding to is the sigma-1 receptor. Under this embodiment "Compound/s binding to the sigma receptor" as used in this application is/are defined as having an IC50 value ≤ 5000 nM (non-claimed), more preferably ≤ 1000 nM (non-claimed), more preferably ≤ 500 nM (non-claimed). More preferably, the IC₅₀ value is ≤ 250 nM (non-claimed). More preferably, the IC₅₀ value is ≤ 100 nM (non-claimed). Most preferably, the IC₅₀ value is ≤ 50 nM. Additionally, the wording "Compound/s binding to the sigma receptor", as used in the present application is defined as having at least ≥ 50% displacement using 10 mM radioligand specific for the sigma receptor (e.g. preferably ¹H-pentazocine).

In another preferred embodiment of the invention (non-claimed), the compound binding to the sigma receptor, as defined above, has an IC₅₀ value of ≤ 1000 nM.

In another preferred embodiment of the invention (non-claimed), the compound binding to the sigma receptor, as defined above, has an IC₅₀ value of ≤ 500 nM.

In another preferred embodiment of the invention (non-claimed), the compound binding to the sigma receptor, as defined above, has an IC₅₀ value of ≤ 250 nM.

In another preferred embodiment of the invention (non-claimed), the compound binding to the sigma receptor, as defined above, has an IC₅₀ value of ≤ 100 nM.

In another preferred embodiment of the invention, the compound binding to the sigma receptor, as defined above, has an IC₅₀ value of ≤ 50 nM.

Most preferably, "compounds highly specific for the sigma receptor" are defined as being "Compound/s binding to the sigma receptor", as defined above, having an IC₅₀ value of ≤ 100 nM (non-claimed).

In a highly preferred embodiment of the present invention, the compound binding to the sigma receptor as defined above, is binding to the sigma-1 receptor subtype.

In another possible aspect of the invention (non-claimed), the compound binding to the sigma receptor as defined above, may bind to the sigma-2 receptor subtype.

In human therapeutics, the dose administered can be quite low depending on the route of administration and is well known in the art because sigma compounds are known therapeutics.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

Any medicament according to the invention contains the active ingredient as well as optionally at least one auxiliary material and/or additive and/or optionally another active ingredient.

The auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

Routes of administration preferably include intramuscular injection, intraveneous injection, subcutaneous injection, sublingual, bucal, patch through skin, oral ingestion, implantable osmotic pump, collagen implants, aerosols or suppository.

Included in this invention (non-claimed) are especially also methods of treatments of a patient or a mammal, including men, suffering from mechanical allodynia using compounds binding to the sigma receptor.

The examples and figures in the following section describing pharmacological trials are merely illustrative and the invention cannot be considered in any way as being restricted to these applications.

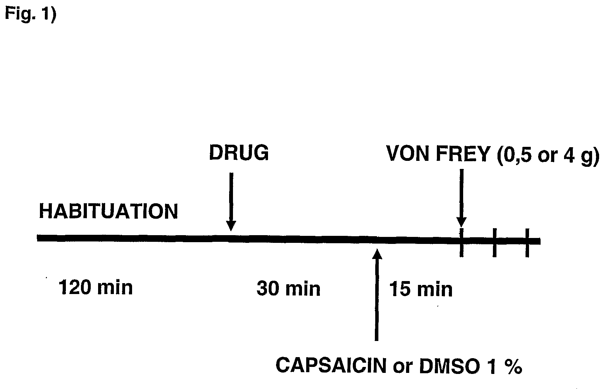

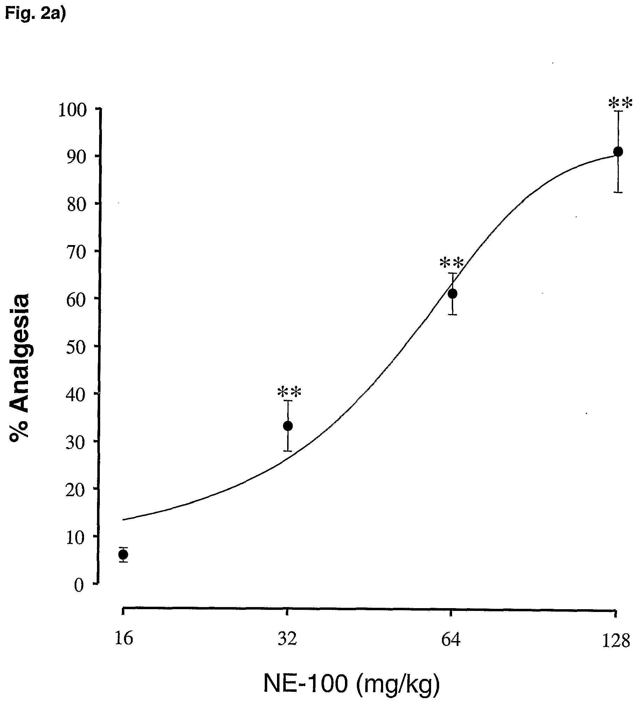

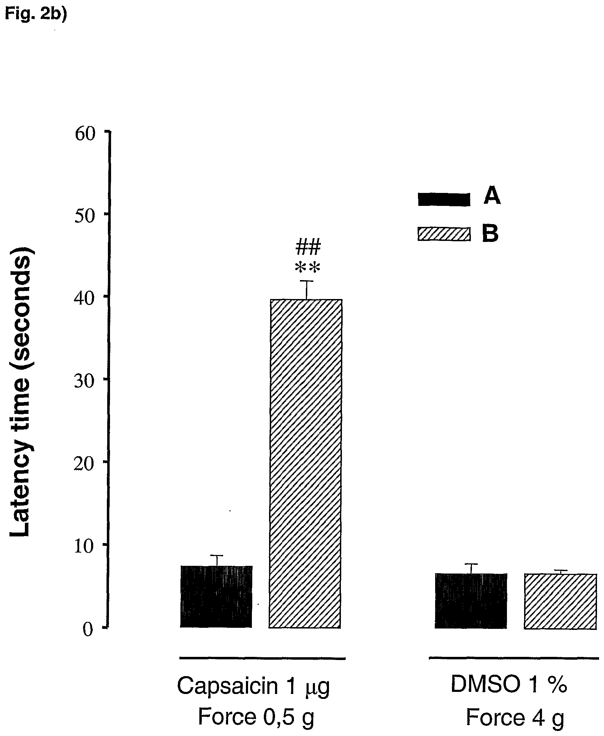

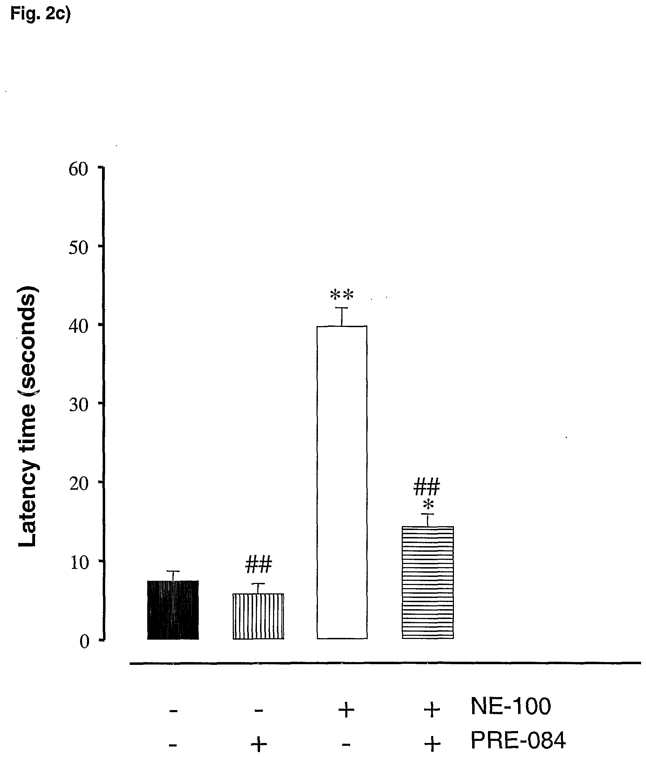

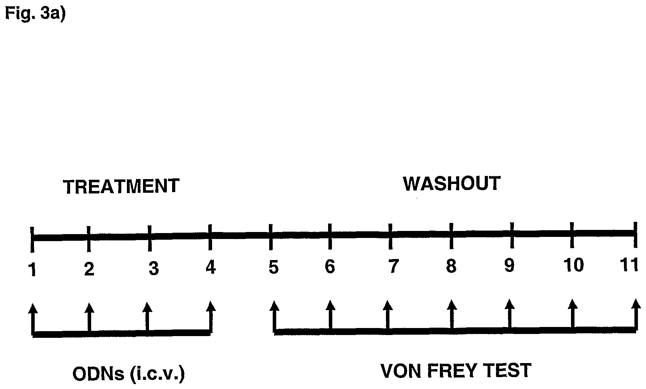

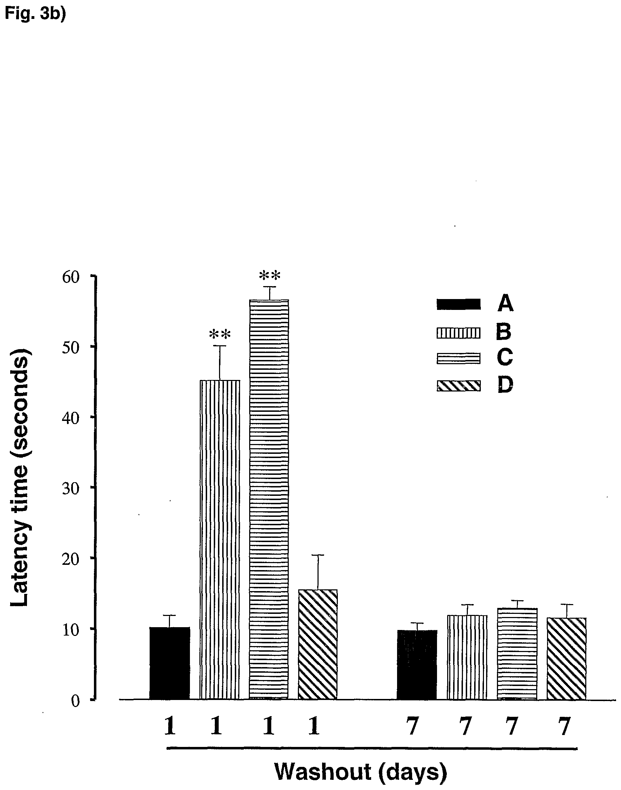

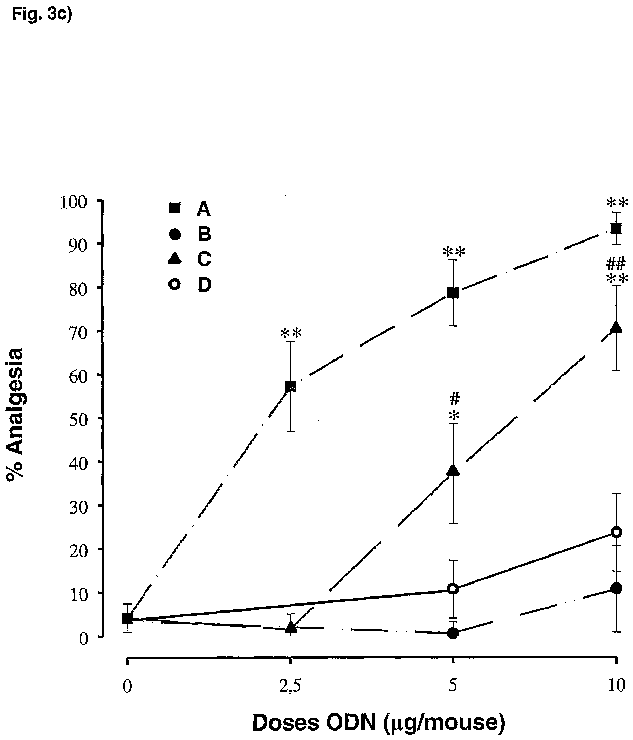

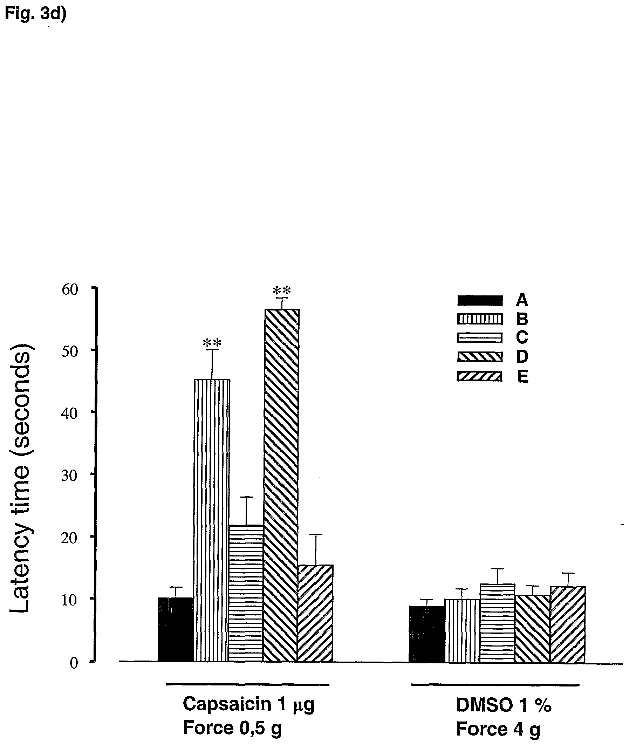

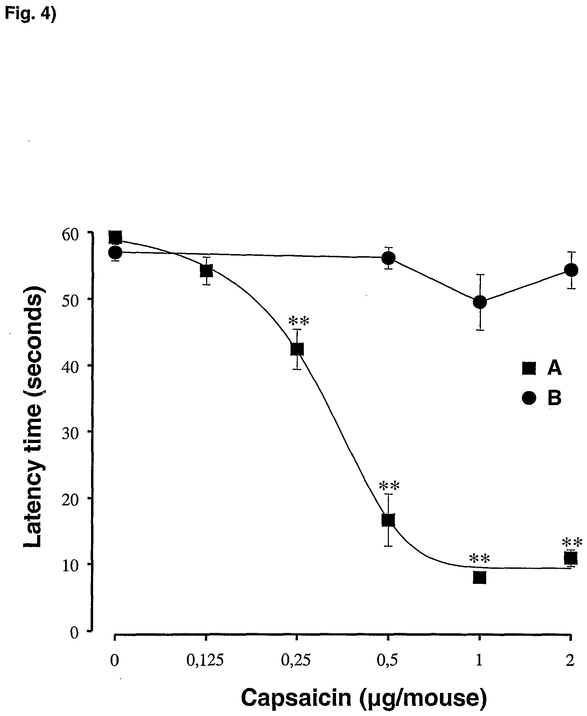

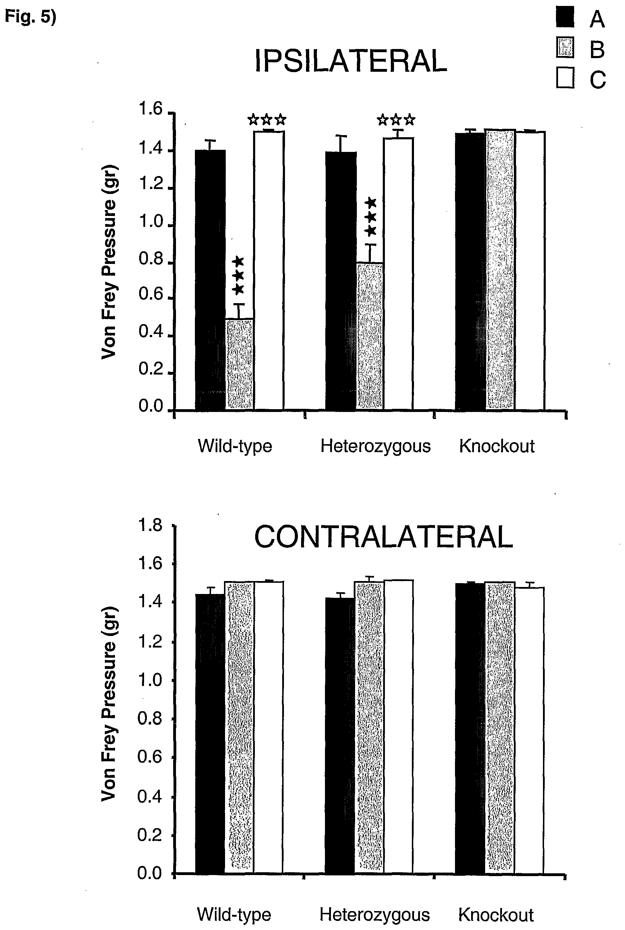

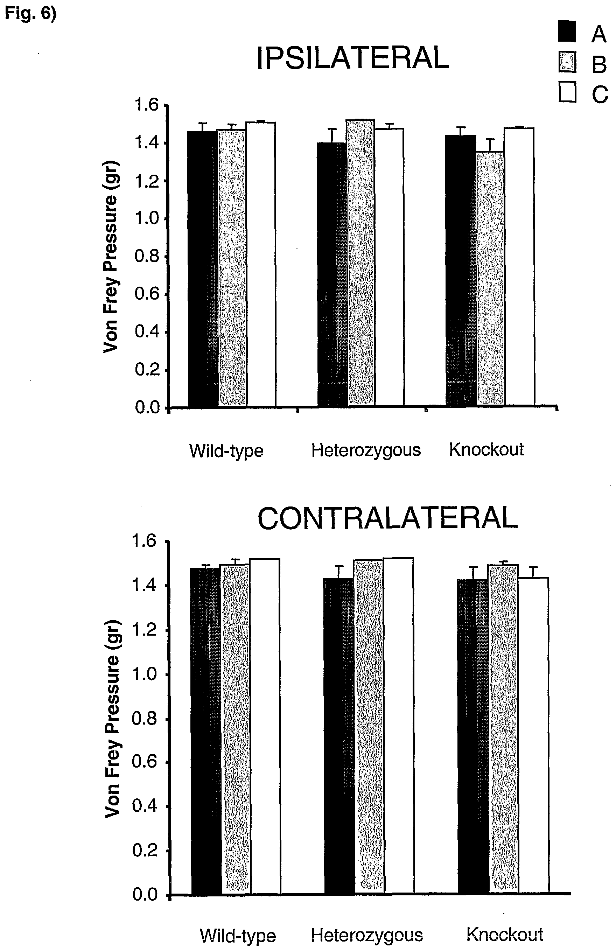

## Claims

1. Use of at least one compound binding to the sigma receptor and having an IC₅₀ value of ≤50 nM for the production of a medicament for the treatment of mechanical allodynia, **characterized in that** said compound binding to the sigma receptor is acting on the sigma receptor as an antagonist, **characterized in that** said compound binding to the sigma receptor is binding to the sigma-1 receptor subtype.

2. Use, according to claim 1, **characterized in that** said compound may be in neutral form, in the form of a base or acid, in the form of a salt, preferably a physiologically acceptable salt, in the form of a solvate and/or in the form of its racemate, pure stereoisomers, especially enantiomers or diastereomers or in the form of mixtures of stereoisomers, especially enantiomers or diastereomers, in any suitable mixing ratio.

3. Use, according to claim 1, **characterized in that** said compound binding to the sigma receptor is selected from the group consisting of:
| | |
|---|---|
| 4-(4-Fluorobenzoyl)-1-(4-Phenylbutyl)Piperidine Oxalate | 4'-Chloro-3-Alpha-(Diphenylmethoxy)Tropane HCl |
| 4-Furan-2-Ylmethyl-Piperazine-1-Carboxylic Acid 2-{4-[3-(2-Trifluoromethyl-Phenothiazin-10-YI)-Propyl]-Piperazin-1-Yl}-Ethyl Ester | Astemizole |
| BD 1008 DiHBr | Benproperine Phosphate |
| BD 1047 | NE 100 |
| BD 1063 | Bromperidol |
| Bromhexine HCl | HEAT HCl |
| Cis-(+/-)-N-Methyl-N-[2-(3, 4-Dichloropheriyl)Ethyl]-2-(1-Pyrrolidinyl)Cyclohexamine DiHBr | GBR 12909 DiHCl |
| GBR-12935 DiHCl | Ifenprodil Tartrate |
| L-693,403 Maleate | Iomerizine HCl |
| MR 16728 HCl | L-741,742 HCl |
| PAPP | Lobeline HCl |
| RS 67333 HCl | Trimeprazine Hemi-L-Tartrate |
| Trifluperidol HCl | |

4. Use, according to claim 1, **characterized in that** said compound binding to the sigma receptor is of general formula IB 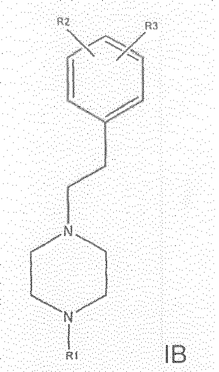 wherein
R1 is selected from C₁₋₆-Alkyl, saturated or unsaturated, substituted or not substituted, branched or not branched;
R2 and R3 are independently of each other selected from H; C₁₋₆-Alkyl, saturated or unsaturated, substituted or not substituted, branched or not branched; Halogen; O-C-₁₋₆ Alkyl, saturated or unsaturated, substituted or not substituted, branched or not branched.

5. Use, according to claim 4, wherein R1 is selected from C₁₋₆-Alkyl, saturated or unsaturated, branched or not branched, unsubstituted or substituted with F, Cl, Br, I₁ NH₂, SH or OH.

6. Use, according to claim 4, wherein R2 and R3 are independently of each other selected from H; C₁₋₆-Alkyl, saturated or unsaturated, branched or not branched, unsubstituted or substituted with F, Cl, Br, I, NH₂, SH or OH; Halogen; O-C₁₋₆-Alkyl, saturated or unsaturated, branched or not branched, unsubstituted or substituted with F, Cl, Br, I, NH₂, SH or OH.

7. Use, according to any of claims 4 to 6, **characterized in** which for the compound according to general formula IB
R¹ is selected from C₁₋₄-Alkyl, saturated or unsaturated, substituted or not substituted, and branched or not branched;
preferably R¹ is selected from C₁₋₄-Alkyl, saturated, substituted or not substituted, and branched or not branched;
more preferably R¹ is selected from C₁₋₄-Alkyl, saturated, not substituted, and branched or not branched; namely CH₃, C₂H₅, C₃H₇, C₄H₉.

8. Use, according to any of claims 4 to 7, **characterized in** which for the compound according to general formula IB
R² and R³ are independently of each other selected from H; OH, SH, NH₂, C₁₋₄- Alkyl, saturated or unsaturated, substituted or not substituted, branched or not branched; F, Cl, Br, I; O-C₁₋₄-Alkyl, saturated or unsaturated, substituted or not substituted, branched or not branched;
preferably R² and R³ are independently of each other selected from H; OH, NH₂, C₁₋₄-Alkyl, saturated, substituted or not substituted, and branched or not branched; F, Cl, Br, I; O-C₁₋₄-Alkyl, saturated, not substituted, and branched or not branched.

9. Use, according to any of claims 4 to 8, **characterized in** which for the compound according to general formula IB
R² and R³ are independently of each other selected from H, OH, NH₂, CH₃, C₂H₅, C₃H₇, C₄H₉, CF₃, CHF₂, Cl, F, Br, I, O-CH₃, O-C₂H₅, O-C₃H₇, O-C₄H₉;
preferably R² and R³ are independently of each other selected from H, F, Cl and CF₃
and/or are in 3' and 4' position respectively.

10. Use according to claim 1, wherein said compound is 1-(3,4-dichlorophenethyl)-4-methylpiperazine.

11. Use according to claim 1, wherein said compound is N1-(3,4-dichlorophenethyl)-N1,N2,N2-trimethylethane-1,2-diamine.

## Patentansprüche

1. Verwendung von mindestens einer Verbindung, die an den Sigma-Rezeptor bindet und einen IC₅₀-Wert von ≤50 nM aufweist, für die Herstellung eines Medikaments zur Behandlung einer mechanischen Allodynie, **dadurch gekennzeichnet, dass** die an den Sigma-Rezeptor bindende Verbindung als Antagonist auf den Sigma-Rezeptor wirkt, **dadurch gekennzeichnet, dass** die an den Sigma-Rezeptor bindende Verbindung an den Sigma-1-Rezeptor-Subtyp bindet.

2. Verwendung nach Anspruch 1, **dadurch gekennzeichnet, dass** die Verbindung in neutraler Form, in der Form einer Lauge oder Säure, in der Form eines Salzes, vorzugsweise eines physiologisch geeigneten Salzes, in der Form eines Solvats und/oder in der Form ihres Racemats, reiner Stereoisomere, insbesondere Enantiomere oder Diastereomere, oder in der Form von Gemischen von Stereoisomeren, insbesondere von Enantiomeren oder Diastereomeren, in einem beliebigen geeigneten Mischungsverhältnis vorliegen kann.

3. Verwendung nach Anspruch 1, **dadurch gekennzeichnet, dass** die an den Sigma-Rezeptor bindende Verbindung aus der Gruppe ausgewählt ist, die aus den Folgenden besteht:
| | |
|---|---|
| 4-(4-Fluorbenzoyl)-1-(4-phenylbutyl)piperidinoxalat | 4'-Chlor-3-alpha-(Diphenylmethoxy)tropan-HCl |
| 4-Furan-2-ylmethyl-piperazin-1-carbonsäure 2-{4-[3-(2-trifluormethyl-phenothiazin-10-yl)-propyl]-piperazin-1-yl}-ethylester | Astemizol |
| BD 1008 DiHBr | Benproperinphosphat |
| BD 1047 | NE 100 |
| BD 1063 | Bromperidol |
| Bromhexin-HCl | HEAT HCl |
| Cis-(+/-)-N-Methyl-N-[2-(3,4-dichlorophenyl)ethyl]-2-(1-pyrrolidinyl)cyclohexamin-DiHBr | GBR 12909 DiHCl |
| GBR-12935 DiHCl | Ifenprodiltartrat |
| L-693,403 Maleat | Iomerizin HCl |
| MR 16728 HCl | L-741,742 HCl |
| PAPP | Lobelin-HCl |
| RS 67333 HCl | Trimeprazinhemi-L-tartrat |
| Trifluperidol HCl | |

4. Verwendung nach Anspruch 1, **dadurch gekennzeichnet, dass** die an den Sigma-Rezeptor bindende Verbindung die allgemeine Formel IB aufweist 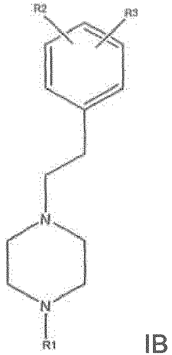 wobei
R1 ausgewählt ist aus C₁₋₆-Alkyl, gesättigt oder ungesättigt, substituiert oder nicht substituiert, verzweigt oder nicht verzweigt;
R2 und R3 unabhängig voneinander ausgewählt sind aus H; C₁₋₆-Alkyl, gesättigt oder ungesättigt, substituiert oder nicht substituiert, verzweigt oder nicht verzweigt; Halogen; O-C-₁₋₆ Alkyl, gesättigt oder ungesättigt, substituiert oder nicht substituiert, verzweigt oder nicht verzweigt.

5. Verwendung nach Anspruch 4, wobei R1 ausgewählt ist aus C₁₋₆-Alkyl, gesättigt oder ungesättigt, verzweigt oder nicht verzweigt, nicht substituiert oder mit F, Cl, Br, I, NH₂, SH oder OH substituiert.

6. Verwendung nach Anspruch 4, wobei R2 und R3 unabhängig voneinander ausgewählt sind aus H; C₁₋₆-Alkyl, gesättigt oder ungesättigt, verzweigt oder nicht verzweigt, nicht substituiert oder mit F, Cl, Br, I, NH₂, SH oder OH substituiert; Halogen; O-C₁₋₆-Alkyl, gesättigt oder ungesättigt, verzweigt oder nicht verzweigt, nicht substituiert oder mit F, Cl, Br, I, NH₂, SH oder OH substituiert.

7. Verwendung nach einem der Ansprüche 4 bis 6, **dadurch gekennzeichnet, dass** in der Verbindung mit der allgemeinen Formel IB
R¹ ausgewählt ist aus C₁₋₄-Alkyl, gesättigt oder ungesättigt, substituiert oder nicht substituiert, verzweigt oder nicht verzweigt;
R¹ vorzugsweise ausgewählt ist aus C₁₋₄-Alkyl, gesättigt, substituiert oder nicht substituiert und verzweigt oder nicht verzweigt;
R¹ insbesondere ausgewählt ist aus C₁₋₄-Alkyl, gesättigt, nicht substituiert und verzweigt oder nicht verzweigt; nämlich CH₃, C₂H₅, C₃H₇, C₄H₉.

8. Verwendung nach einem der Ansprüche 4 bis 7, **dadurch gekennzeichnet, dass** in der Verbindung mit der allgemeinen Formel IB
R² und R³ unabhängig voneinander ausgewählt sind aus H; OH, SH, NH₂, C₁₋₄-Alky, gesättigt oder ungesättigt, substituiert oder nicht substituiert, verzweigt oder nicht verzweigt; F, Cl, Br, I; O-C₁₋₄-Alkyl, gesättigt oder ungesättigt, substituiert oder nicht substituiert, verzweigt oder nicht verzweigt;
R² und R³ vorzugsweise unabhängig voneinander ausgewählt sind aus H; OH, NH₂, C₁₋₄-Alkyl, gesättigt, substituiert oder nicht substituiert und verzweigt oder nicht verzweigt; F, Cl, Br, I; O-C₁₋₄-Alkyl, gesättigt, nicht substituiert und verzweigt oder nicht verzweigt.

9. Verwendung nach einem der Ansprüche 4 bis 8, **dadurch gekennzeichnet, dass** in der Verbindung mit der allgemeinen Formel IB
R² und R³ unabhängig voneinander ausgewählt sind aus H, OH, NH₂, CH₃, C₂H₅, C₃H₇, C₄H₉, CF₃, CHF₂, Cl, F, Br, I, O-CH₃, O-C₂H₅, O-C₃H₇, O-C₄H₉;
R² und R³ vorzugsweise unabhängig voneinander ausgewählt sind aus H, F, Cl und CF₃
und/oder sich in 3'- bzw. 4'-Position befinden.

10. Verwendung nach Anspruch 1, wobei es sich bei der Verbindung um 1-(3,4-Dichlorphenethyl)-4-methylpiperazin handelt.

11. Verwendung nach Anspruch 1, wobei es sich bei der Verbindung um N1-(3,4-Dichlorphenethyl)-N1,N2,N2-trimethylethan-1,2-diamin handelt.

## Revendications

1. Utilisation d'au moins un composé se liant au récepteur sigma et possédant une valeur IC₅₀ inférieure ou égale à 50 nM pour la production d'un médicament pour le traitement de l'allodynie mécanique, **caractérisée en ce que** ledit composé se liant au récepteur sigma agit sur le récepteur sigma en tant qu'antagoniste, **caractérisée en ce que** ledit composé se liant au récepteur sigma se lie au sous-type de récepteur sigma-1.

2. Utilisation selon la revendication 1, **caractérisée en ce que** ledit composé peut-être dans une forme neutre, sous la forme d'une base d'un acide, sous la forme d'un sel, de préférence d'un sel physiologiquement acceptable, sous la forme d'un solvate et/ou sous la forme de son racémate, de ses stéréoisomères pures, spécialement des énantiomères ou des diastéréoisomères ou sous la forme de mélanges de stéréoisomères, spécialement des énantiomères ou des diastéréoisomères, dans n'importe quel rapport de mélange convenable.

3. Utilisation selon la revendication 1, **caractérisée en ce que** ledit composé se liant au récepteur sigma est choisi parmi le groupe constitué de :
| | |
|---|---|
| Oxalate de la 4-(4-Fluorobenzoyl)-1-(4-Phénylbutyl)Pipéridine | 4'-Chloro-3-Alpha-(DiPhénylméthoxy)Tropane HCl |
| Acide Carboxylique de l'Ester de 2-{4-[3-(2-Trifluorométhyl-Phénothiazin-10-Yl)-Propyl]-Pipérazin-1-Yl}-Éthyle | Astémizole |
| BD 1008 DiHBr | Phosphate de Benpropérine |
| BD 1047 | NE 100 |
| BD 1063 | Brompéridol |
| Bromhexine HCl | HEAT HCl |
| Cis-(+/-)-N-Méthyl-N-[2-(3, 4-DichloroPhényl)Éthyl]-2-(1-Pyrrolidinyl)Cyclohexamine DiHBr | GBR 12909 DiHCl |
| GBR-12935 DiHCl | Tartrate d'Ifenprodil |
| L-693,403 Maléate | Iomerizine HCl |
| MR 16728 HCl | L-741,742 HCl |
| PAPP | Lobéline HCl |
| RS 67333 HCl | Hémi-L-Tartrate de Triméprazine |
| Triflupéridol HCl | |

4. Utilisation selon la revendication 1, **caractérisée en ce que** ledit composé se liant au récepteur sigma est de formule générale IB 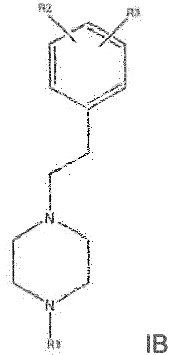 dans laquelle
R¹ est choisi parmi C₁₋₆-Alkyle, saturé ou insaturé, substitué ou non substitué, ramifié ou non ramifié ;
R² et R³ sont indépendamment l'un de l'autre choisis parmi H ; C₁₋₆-Alkyle, saturé ou insaturé, substitué ou non substitué, ramifié ou non ramifié ; halogéne ; O-C₁₋₆-Alkyle, saturé ou insaturé, substitué ou non substitué, ramifié ou non ramifié.

5. Utilisation selon la revendication 4, dans laquelle R¹ est choisi parmi C₁₋₄-Alkyle, saturé ou insaturé, ramifié ou non ramifié, non substitué ou substitué avec F, CI, Br, I, NH₂, SH ou OH.

6. Utilisation selon la revendication 4, dans laquelle R² et R³ sont indépendamment l'un de l'autre choisis parmi H ; C₁₋₆-Alkyle, saturé ou insaturé, ramifié ou non ramifié, non substitué ou substitué avec F, CI, Br, I, NH₂, SH ou OH ; halogène ; O-C₁₋₆-Alkyle, saturé ou insaturé, ramifié ou non ramifié, non substitué ou substitué avec F, CI, Br, I, NH₂, SH ou OH.

7. Utilisation selon l'une quelconque des revendications 4 à 6, **caractérisée en ce que** pour le composé selon la formule générale IB
R¹ est choisi parmi C₁₋₄-Alkyle, saturé ou insaturé, substitué ou non substitué, ramifié ou non ramifié ;
de préférence R¹ est choisi parmi C₁₋₄-Alkyle, saturé ou insaturé, substitué ou non substitué, et ramifié ou non ramifié ;
plus préférablement R¹ est choisi parmi C₁₋₄-Alkyle, saturé ou insaturé, substitué ou non substitué, et ramifié ou non ramifié ; à savoir CH₃, C₂H₅, C₃H₇, C₄H₉.

8. Utilisation selon l'une quelconque des revendications 4 à 7, **caractérisée en ce que** pour le composé selon la formule générale IB
R² et R³ sont indépendamment l'un de l'autre choisis parmi H ; OH, SH, NH₂, C₁₋₄-Alkyle, saturé ou insaturé, substitué ou non substitué, ramifié ou non ramifié ; F, CI, Br, I ; O-C₁₋₄-Alkyle, saturé ou insaturé, substitué ou non substitué, ramifié ou non ramifié ;
de préférence, R² et R³ sont indépendamment l'un de l'autre choisis parmi H ; OH, NH₂, C₁₋₄-Alkyle, saturé ou insaturé, substitué ou non substitué, ramifié ou non ramifié ; F, CI, Br, I ; O-C₁₋₄-Alkyle, saturé ou insaturé, substitué ou non substitué, ramifié ou non ramifié.

9. Utilisation selon l'une quelconque des revendications 4 à 8, **caractérisée en ce que** pour le composé selon la formule générale IB
R² et R³ sont indépendamment l'un de l'autre choisis parmi H, OH, NH₂, CH₃, C₂H₅, C₃H₇, C₄H₉, CF₃, CHF₂, CI, F, Br, I, O-CH₃, O-C₂H₅, O-C₃H₇, O-C₄H₉ ;
de préférence, R² et R³ sont indépendamment l'un de l'autre choisis parmi H, F, CI et CF₃
et/ou sont respectivement en position 3' et 4'

10. Utilisation selon la revendication 1, dans laquelle ledit composé est la 1-(3,4-dichlorophénéthyl)-4-méthylpipérazine.

11. Utilisation selon la revendication 1, dans laquelle ledit composé est la N1-(3,4-dichlorophénéthyl)-N1,N2,N2-triméthyléthane-1,2-diamine.
